(19) Europäisches Patentamt
European Patent Office
Office européen des brevets

(11) Publication number : **0 534 772 A2**

# (12) EUROPEAN PATENT APPLICATION

(21) Application number : **92308742.3**

(51) Int. Cl.$^5$ : **C11D 3/39,** D06L 3/02

(22) Date of filing : **25.09.92**

(30) Priority : **27.09.91 GB 9120644**

(43) Date of publication of application : **31.03.93 Bulletin 93/13**

(84) Designated Contracting States : **PT**

(71) Applicant : **WARWICK INTERNATIONAL GROUP LIMITED**
**Wortley Moor Road**
**Leeds LS12 4JE (GB)**

(72) Inventor : **Townend, John**
**27 The Beeches**
**Holywell, Clwyd (GB)**
Inventor : **Crowther, Jan Darrel**
**3 Vaughn Close Prestatyn**
**Clwyd LL19 8SH (GB)**

(74) Representative : **Jones, Helen Marjorie Meredith**
**Gill Jennings & Every, Broadgate House, 7 Eldon Street**
**London EC2M 7LH (GB)**

## (54) Oxidising compositions.

(57) A polyacid polyanhydride is used as a bleach activator especially for improving low temperature bleaching performance of peroxygen bleaches such as hydrogen peroxide or sodium perborate, in laundry and other detergent formulations. The polyanhydrides are preferably made from polybasic, usually dibasic, carboxylic acids, which may be aliphatic and/or aromatic in nature. The polyanhydride may be a homopolymer or a copolymer and has at least 2 monomer units on average per chain. The polyanhydride is preferably made by reacting the free dibasic acids with excess acetic anhydride and refluxing until the desired molecular weight is achieved followed by removal of acetic acid formed and excess acetic anhydride. Examples of polyanhydrides are polymers of sebacic, adipic, azelaic, succinic, fumaric, iso- and per-phthalic and citric acids. The polyanhydrides are solids which are easy to handle, relatively storage stable and give bleach activation.

EP 0 534 772 A2

Jouve, 18, rue Saint-Denis, 75001 PARIS

The present invention relates to the new use of anhydride compounds as bleach activators, useful in compositions in combination with inorganic or organic peroxygen releasing compounds, to new percarboxylic bleaching agents and to new anhydrides useful as bleach activators, as well as bleaching compositions containing such compounds and processes for producing the compounds.

It is known that percarboxylic acid compounds are useful as bleaching agents, general oxidising agents and biocides. Such compounds may be incorporated as such into bleaching compositions, for instance laundry detergents, hard surface cleaners, paper and pulp bleaching compositions, fabric and fibre bleaching compositions and even depilatory creams as well as biocidal compositions, for use in agriculture.

In many references the peracids are produced from the corresponding anhydrides, including anhydrides of dibasic acids in organic solvent using hydrogen peroxide. For instance in US-A-3,365,487 the production of mono percarboxylic acid derivatives from intra molecular anhydrides of dibasic cycloaliphatic and aromatic carboxylic acids in organic solvent is described. In US-A-3,510,512 a similar process for producing mono perphthalic acid from phthalic anhydride is disclosed and in US-A-4,085,133 monoperoxyphthalic acid is produced from phthalic anhydride

In US-A-3,655,738 diperphthalic acids, in particular diperisophthalic acid is formed and in US-A-4134850 monoand polyperacid derivatives of cyclohexyldi- and multi-carboxylic acid compounds are described.

It is known that organic peracids can be dangerous to handle and various ways of overcoming these problems have been described. For instance in US-A-3,770,816 diperisophthalic acids are stabilised (against detonation or explosion) by the incorporation of inorganic hydrated salt, especially magnesium and aluminium salts.

In EP-A-0,027,693 magnesium salts of monoperoxydicarboxylic aromatic or cycloaliphatic acids are produced from the corresponding intra molecular anhydrides in the presence of magnesium oxide or magnesium carbonate. In EP-A-0,066,992 hydrated magnesium salts of monoperoxy dicarboxylic acids, including -phthalic and -maleic acid are described, which are produced by a similar process. In EP-A-0,074,730 particles of the magnesium salts of the monoperoxydicarboxylic acids are granulated by spraying the particles whilst mixing with an aqueous solution of a hydroxylated organic polymer, for instance polyvinyl alcohol (PVA) polymer. Other examples of attempts to stabilise peracids in bleaching compositions are given in EP-A-0,373,691, EP-A-0,254,331, EP-A-0,256,443, EP-A-0,206,740, EP-A-0,360,223.

In SU-A-825518 monoperphthalic acid is produced by reacting phthalic anhydride with hydrogen peroxide in the presence of zinc oxide, cadmium oxide or magnesium oxide catalyst and an alkane phosphonic acid as stabiliser. The product is used as an oxidising agent in the synthesis of epoxides, as a PVC stabiliser and a bleach, for fibres of wood and paper.

In US-A-2,813,896 and US-A-4,259,201 $\alpha,\omega$-long chain aliphatic diperoxoic acids are described as bleaching agents. Preferred compounds are diperoxy dodecanedioic acid, diperoxy azelaic acid and diperoxy adipic acid.

In EP-A-0,083,560 2-substituted-butane-1,4-diperoxy acids are disclosed. The 2-substituent is preferably C8-14 straight chain alkyl. One of the comparative examples compares decylbutanediperoxoic with $\alpha,\omega$-dodecanediperoxoic acid for bleaching performance.

In EP-A-0,136,280 substituted butane diperoxoic acids are formed by peroxidising substituted succinic anhydride. The substituent is usually an alkyl group. Other succinic peracid derivatives are described in EP-A-0,186,650, EP-A-0,179,223, EP-A-0,168,587.

In GB-A-2,129,454 compositions containing monoperoxyphthalic acid or salts are combined with metal complexing agents such as alkane phosphonic acids, inorganic persalts and phthalic anhydride activator in a bleaching composition.

In EP-A-168,204 a monoperoxy alkylsuccinic acid is formed by reacting the corresponding the succinic anhydride with hydrogen peroxide in the presence of one water-immiscible and one water-miscible organic solvent.

In DD-A-272,654 percarboxylic acid derivatives of copolymers of ethylenically unsaturated dicarboxylic acids with ethylene are used as bleaching compounds. The peracids are produced by the heterogeneous reaction of the copolymeric anhydride with hydrogen peroxide in the absence of solvents and catalysts, and in the optional presence of basic alkali metal or alkaline earth metal compound in order to produce the salt is required.

In US-A-4,473,507 monoperoxycarboxylic acids, for instance monoperoxydicarboxylic acids, or salts are used in combination with an anionic surfactant, and with an acid having an pKa of 2 to 7, preferably 3 to 5 as a controlled bleach release composition for laundry washing. Suitable acids are benzoic, adipic, succinic, citric, tartaric and glutaric.

Various nitrogen-containing peroxycarboxylic acids have also been described for use as bleaching agents. The percarboxylic acids may themselves be incorporated into detergent compositions or in some disclosures

may be formed in situ by combining a corresponding bleach activator which comprises a leaving group in place of the -OOH group which reacts in situ with persalts in the bleaching composition.

Most of the above references describe the incorporation of peracids into granular or other bleaching or detergent compositions in the peracid or salt form. It is also known that carboxylic peracids can be formed in situ in the wash or bleaching liquor by the reaction of a precursor, or activator and inorganic persalt, such as a percarbonate, persulphate or, most often a perborate. Activators are acyl-releasing agents. Many types of activators are known and are usually amido or anhydride derivatives of acid, phenyl esters, alkenyl esters etc. It has been described in many instances that intramolecular dibasic anhydrides such as succinic anhydride, maleic anhydride and phthalic anhydride and substituted derivatives of these can be used as bleach activators. In GB-A-2,193,510 a mixture of phthalic anhydride and maleic and/or succinic anhydride is used as bleach activator. The mixture comprises a higher amount of phthalic anhydride than maleic and succinic.

GB-A-2,205,867 describes the use of an intramolecular anhydride, of 5-norbornene-2,3-dicarboxylic acid as a bleach activator. In EP-A-0,331,300 cyclic anhydrides are described which include in the anhydride ring a nitrogen atom adjacent one of the carboxyl groups, for instance isatoic anhydride, used as a bleach activator, optionally in combination with other bleach activators such as phthalic, maleic or succinic anhydride.

Mixed anhydrides are used as bleach activators in US-A-3,338,839. The activators are either co-anhydrides of an aliphatic monocarboxylic acid and an aromatic mono- or poly-carboxylic acid, in which any additional carboxylic acid group is not ortho to the first acid group or are admixtures of homo anhydrides of the aliphatic carboxylic mono acid and of the homo anhydride of the mono or poly aromatic acids. The suitable poly basic aromatic acids for use in either aspect are for instance isophthalic or perphthalic acid. It is disclosed that where the activator is a coanhydride of a di- or poly-basic aromatic carboxylic acid the additional carboxylic acid groups of the aromatic compound may be combined with additional aliphatic carboxylic acid to form eg the diacetic anhydride of terephthalic acid. It is asserted that the physical and chemical mixed anhydrides of the stated aliphatic and aromatic acids gives a synergistic improvement in bleaching properties. The coanhydride of a monobasic aromatic acid with acetic acid gave equivalent results to the physical admixture of the homoanyhdrides of the same acids. One example uses the diacetic anhydride of isophthalic acid. The coanhydrides are made by reacting acetic anhydride with the aromatic acid though there are no specific details of the reaction conditions for the production of any of the exemplified coanhydrides. Furthermore comparative examples using intramolecular anhydrides of dibasic aliphatic carboxylic acids show poor bleaching activities. Anhydrides of sulphonic acids have also been described as bleach activators in US-A-4,111,651 and US-A-4,165,805. The compounds described may be symmetrical or unsymmetrical anhydrides of monobasic aliphatic and aromatic sulphonic acids.

In US-A-4,110,074 mixed anhydrides of sulphonic and carboxylic acids are described. The acids may be aromatic or aliphatic (including cycloaliphatic) in nature and may be 1-3 valent (in terms of acid groups present) but where polyvalent, apparently all of the acid groups must be of the same type. The anhydrides are made by mixing the silver salt of the sulphonic acid with the acid chloride of the carboxylic acids so that homoanhydride groups cannot be formed. There are specific examples of the production and use of terephthaloyl-bis (p-toluenesulphonate) isophthaloyl-bis (p-toluene-sulphonate) and isophthaloyl-bis (methanesulphonate), but the properties of those compounds compared to the mixed anhydrides formed from the mono basic acids seem to be worse.

Esters of carboxylic acids with phenols are also known as bleach activators. In EP-A-0,252,724 unsymmetrical diesters of dihydroxy benzene are disclosed as activators for peroxygen bleaches. The diesters are produced by selectively monoacylating dihydroxybenzenes using an acid anhydride, such as octanoic anhydride. The monoester is then further acylated using a second acid anhydride, such as acetic anhydride. The use of one relatively hydrophilic acyl group and one relatively hydrophobic acyl group is disclosed as giving a good combination of bleaching activity by reacting with less or more oily stains.

In JP-A-60-222071 luminescent balls for night-time golf playing comprise separate compartments one of which contains a) a hydroperoxide, another of which contains b) a derivative of oxalic acid that can be a dianhydride with a monobasic carboxylic acid such as acetic, triphenylacetic, lauric or p-methoxybenzoic acid, or that can be a dianhydride of a dibasic carboxylic acid

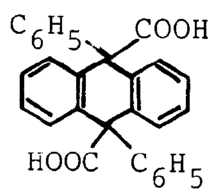

and another compartment of which contains c) a fluorescent compound, and optionally water catalysts and/or diluent in one or a different compartment. The components are allowed to mix when the ball is hit to rupture the compartments and the chemical reaction is said to generate light. There is no further explanation of the reaction. In US-A-3,804,891 and J. Am Chem Soc (1967) 89, 6523 Bollyky et al describe a similar chemiluminescent reaction in which oxalic acid anhydrides react with hydrogen peroxide in the presence of fluorescent compounds. The anhydrides tested are bi's (triphenylacetic)-, diacetic-, bis(p-methoxybenzoic)- and dibenzoic oxalic anhydrides. The reaction was carried out in non aqueous solvent, though the addition of small amounts of water was investigated. The reaction mechanism is proposed to involve the cleavage of one of the anhydride groups by hydrogen peroxide to form a monoperoxy oxalic acid which subsequently reacts with 9, 10-diphenylanthracene with emission of carbon dioxide and carbon monoxide to excite the fluorescer. The dianhydrides are made by reaction of the potassium salt of the substituted acetic or benzoic acid with oxalylchloride under anhydrous conditions or by reaction of ketene with anhydrous oxalic acid. The products were all stated to have limited stability against hydrolysis and thermal decomposition.

Polyanhydrides produced by reacting dibasic carboxylic acids or derivatives in reactions which will lead to chains comprising anhydride linkages are known. Carothers in J. Am. Chem. Soc. (1930) 52, 3470-1 explains that adipic anhydride produced by the usual methods is not monomeric but polymeric and that the polymeric form can be converted to the monomeric cyclic form by heating in vacuo. Hill in J. Am. Chem. Soc (1930) 52, 4100-14 describes the formation of adipic anhydride by two methods. The first refluxes adipic acid in excess acetic anhydride for 4-6 hours, followed by removal of excess acetic anhydride and acetic formed and recovery of the solid. The second method reacts adipic acid with acetyl chloride. Both methods are said to produce polymeric adipic acid.

In the above two references and in J. Am. Chem. Soc. (1933)55, 5023-31, Hill and Carothers describe the depolymerisation of adipic anhydride polymer to the monomeric form, which spontaneously repolymerises especially in the presence of water. The polymeric form, they found to have a molecular weight of about 5000 and could polymerise further to a super polymeric form with a molecular weight about 10,000 under the same conditions as depolymerisation to form monomeric adipic anhydride, these being heating under high vacuum with a condenser being placed close to the evaporating surface. Similar reactions of the $C_{7-18}$ analogs are also described. No uses are suggested for the polymer products.

In J. Macromol. Sci., Chem., (1990), A27 (4), 397-412 Albertsson et al describe a ring opening polymerisation of adipic anhydride in the presence of acetic anhydride catalysed by stannous 2-ethylhexanoate. The product is subsequently depolymerised to form oxepane-2,7-dione. In DE-A-1804067 anhydrides of monobasic and dibasic carboxylic acids are formed in 1-step reaction of molten acids with gaseous ketene. When dibasic acids are used for instance sebacic, it is stated that the product will include polymeric anhydrides, with intermolecular anhydride groups. No uses for the product are disclosed.

A large amount of work has recently been carried out by Langer, Leong, Domb et al at the Massachusett's Institute of Technology. In Macromolecules 20(4), (1987) 705-712 various methods of producing polyanhydrides are described. Cyclic anhydrides are condensed by refluxing a melt with a corresponding acid. The polymers had average molecular weights of at least 50,000, when the process was carried out for more than 24 hours. Another method involves reacting acyl chlorides with carboxylic acids in a dehydrohalogenation reaction in an interfacial hexane-DMF solvent. The polymer produced by that method had a degree of polymerisation between 15 and 30. Another process involved the dehydrative coupling of the acids, using dehydrative coupling agents including N, N-bis[2-oxo-3-oxazolidinyl] phosphoramidoyl chloride or phenyl-N-phosphonamidoyl chloride. The degree of polymerisation of the polymer products was again 15-30.

In WO-A-8900855 the dicarboxylic acids used to produce polymers have one aryl carboxylic acid group and one aliphatic carboxylic acid group in a monomer molecule, for instance p-carboxyphenoxyvaleric acid and p-carboxyphenoxyoctanoic acid. In WO-A-8901005 dicarboxylic acids in an inert solvent, in the presence of an insoluble base are reacted with a coupling agent, for instance phosgene, diphosgene or an acyl chloride. The monomers may have amide groups separating the two carboxylic acid groups, so that the product is a poly(amide-anhydride). In US-A-4,886,870 polymers formed from a mixture of two different dibasic acids or their anhydrides were formed. For instance a copolymer of bis(p-carboxyphenoxy) propane anhydride and sebacic acid is formed, by refluxing with acetic anhydride to form a pre-polymer followed by melt-condensation under nitrogen. In US-A-4,999,417, polymerisation of dicarboxylic units based on modified amino acids are homo or co-polymerised by a melt polymerisation technique or in solution.

In US-A-4,868,265 a polyanhydride is formed by condensing a bis(trimethylsilyl) ester of a dicarboxylic acid with a dicarboxylic chloride to give the polyanhydride and chlorotrimethylsilane as a by-product.

The polyanhydride polymers described by the Langer group are used as physical matrices for controlled release of active ingredients. The polymers hydrolyse over a period of time in an aqueous environment, but

the polymers and their biodegraded products are found to be non-toxic. The controlled release formulations are for instance for pharmaceuticals, pesticides, herbicides, fungicides.

In GB-A-2,185,979 it is disclosed that the types of dibasic acids from which polyanhydrides are formed affects their hydrolytic stability. For instance aliphatic polyanhydrides are said to be too readily hydrolysed for use as a matrix for pharmacologically active agents. Purely aromatic polyanhydrides are said to be very stable but insoluble even in organic solvents.

Another use which has been described for polyanhydride polymers where the polymer undergoes a chemical reaction is as a crosslinker/curing agent for epoxy resins. Examples of such disclosures are in GB-A-1,074,293 and GB-A-1,424,966. In JP-A-60-141725 it is disclosed that polyacid polyanhydrides obtained using either decane-1,10-dicarboxylic acid or octadecane-1,18-dicarboxylic acid are preferred to the anhydrides formed from azelaic or sebacic acid by being less readily hydrolysed and so having better stability, curing properties (of epoxy resins) and being readily obtained in powder form. The number of units in a polymer molecule is said to be in the range 1 to 30, usually 3 to 7.

In EP-A-299,420 epoxy resin based powder coating compositions contain a curing agent comprising a polyol-modified polymeric polyanhydride, for instance formed of adipic, azaleic, sebacic or dodecanedioic acids.

In the invention there is provided a new process in which a polyacid polyanhydride compound comprising at least 2 polyacid units per molecule is reacted with a peroxygen compound to cleave an anhydride linkage in aqueous solution to form a peracid compound and the peracid compound is used as an oxidising agent.

The peracid compound is usually used immediately as an oxidising agent, that is the formation of the peracid is carried out in situ, usually in the presence of the substrate to be oxidised, without any intermediate recovery or purification. The process is of most use where the oxidation reaction is a bleaching process and the polyanhydride thus acts as a bleach activator to allow bleaching to be carried out at low temperatures.

According to a further aspect of the invention there is provided a new use of a polyacid polyanhydride comprising at least 2 polyacid units per molecule as a peroxygen bleach activator as well as a new bleaching composition comprising a polyacid polyanhydride and optionally a peroxygen bleach precursor.

The polyanhydride compound is formed from monomeric units which are derived from polybasic acids joined by anhydride linkages in the backbone. The polyanhydride compound may be a homopolymer, that is formed from one polyacid monomer, or may be formed from a mixture of monomers in which case it is a copolymer. The acid monomers usually comprise two acid moieties per molecule although may comprise three or more acid moieties per molecule.

The acid moieties of the units preferably include carboxylic acid moieties although may additionally, or alternatively, include sulphonic acid or phosphonic acid units. The anhydride linkages may be mixed, that is be derived from two different classes of acids, for instance carboxylic and sulphonic acids. Preferably the anhydride linkages are derived from two of the same acid moieties, preferably from two carboxylic acid moieties.

Preferably the acid moieties of a monomer unit are not joined immediately to one another, but are preferably separated by at least one and preferably more than one carbon atom. Thus for instance, it is preferred that a dibasic carboxylic acid is not oxalic acid.

Molecules of the polyanhydride should preferably comprise on average at least 2, preferably at least 5, more preferably at least 10 or more than 20 monomer units on average.

Preferably in the invention the anhydride has the general formula I:

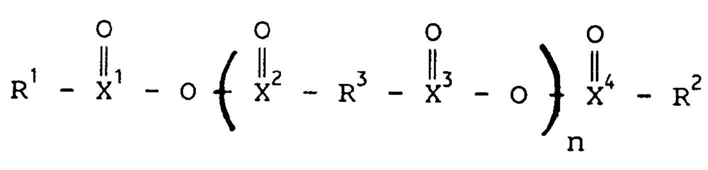

(I)

in which $R^1$ and $R^2$ are the same or different and each represents hydrogen, hydroxy, OM, halogen optionally substituted alkyl, optionally substituted alkenyl, optionally substituted alkynyl or optionally substituted aryl.

$X^1$ to $X^4$ are the same or different and each represents a carbon atom, a sulphur atom

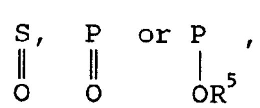

in which $R^5$ is hydrogen a metal ion or an optionally substituted alkyl group,

n is an integer of 2 or more,

the or each $R^3$ is independently selected from a bond, optionally substituted alkylene, optionally substituted alkenylene, optionally substituted alkynylene, optionally substituted arylene in any of which the $R^3$ group may be interrupted by a group selected from

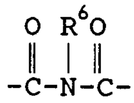 , 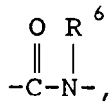 , 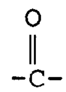 ,

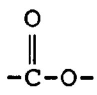 , -O-, 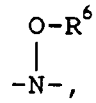 , -S-, 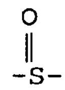 , 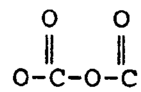 , 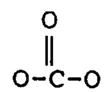 ,

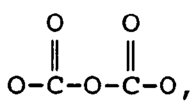 , 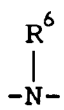 , 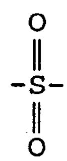 , -S-O-, 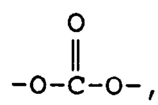 , 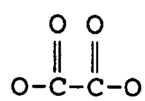 ,

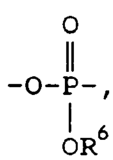 , 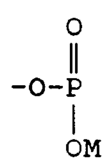 , 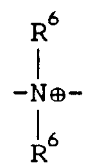

or a linear polyphosphate in which the or each $R^6$ is independently selected from hydrogen, optionally substituted alkyl, optionally substituted alkenyl, optionally substituted alkynyl and optionally substituted aryl

the or each M is a monovalent metal ion.

In the formula n is preferably at least 5 more preferably at least 10 or more than 20. It is preferably less than 50.

In the compound of the formula I preferably $X^1$ to $X^4$ are each selected from a carbon atom,

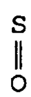 and 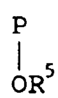 ,

and preferably at least 1 of $X^1$ to $X^4$ is a carbon atom.

Thus the anhydride units in this further aspect of the invention are preferably dicarboxylic acid units, although the polymeric anhydride may also comprise other anhydride units in its backbone, for instance sulphonic acid anhydride units, phosphonic acid anhydride units or mixed units of either of these types of acids or with carboxylic acid.

The polymeric anhydrides include macrocylic anhydrides, that is which include two or more dibasic acid units, the ends of the polymer chain i.e. $R^1$ and $R^2$ of which can be joined to form a macrocyclic molecule.

In the compound of the formula I each group $R^3$ is preferably a group in which the moieties $X^2$ and $X^3$ are separated from one another so as to make formation of cyclic intramolecular anhydride

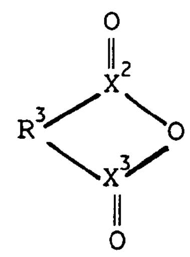

energetically unfavourable.
Preferably therefore $R^3$ is not arylene with $X^2$ and $X^3$ substituted ortho to one another and is not (cis)1,2-ethenylene, 1,2-ethylene, 1,3-propylene or methylene or derivatives thereof or cyclo alkylene with $X^2$ and $X^3$ substituted on adjacent carbon atoms in cis relationship.

In the compound of the formula I at least one of the groups $R^3$ is preferably $C_{2-30}$-alkylene group. Preferably the polymeric anhydride comprises at least two different groups $R^3$ preferably having different hydrophilicity. Preferably at least one of the groups $R^3$ is a $C_{2-5}$-alkylene group and at least one other $R^3$ is a $C_{6-30}$-alkylene group or an arylene group. The two types of alkylene chain thus have different degrees of hydrophilicity.

In compounds in which there are at least two different groups $R^3$ or in which the X groups of an anhydride linking group are different, the anhydride link tends to be more subject to hydrolysis since the asymmetry of the bond tends to destabilise the linkage. Where there is such asymmetry the compound may therefore be advantageously reactive in the perhydrolysis reaction of the invention.

The terminating groups $R^1$ and $R^2$ of the polymer chain may comprise the free acid or metal salt forms of the polyacid monomer units from which the polyanhydride is formed. It is often preferred for the groups to be derived from monobasic acids, that is

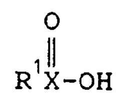

and

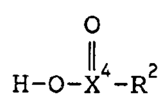

in which $R^1$ and $R^2$ are different or, preferably, same and each represent an alkyl group or an aryl group, each of which may be substituted. In a particularly preferred class of compounds $R^1$ and $R^2$ are the same and are lower alkyl, more preferably methyl. In another preferred class of compounds $R^1$ and $R^2$ are the same and are higher (i.e. greater than $C_5$) alkyl or aryl. It has been found that the type of terminating groups can affect the stability or hydrolytic reactivity under storage and use conditions, and an appropriate terminating group can be determined by experiment.

In all aspects of the invention any alkyl groups may be branched or straight chain alkyl or may include cycloalkyl or saturated heterocyclic groups. Any aryl groups include heteroaryl. Optional substituents on alkyl, alkenyl, alkynyl or aryl groups include halogen, nitro, amine including primary and secondary alkyl or aryl amine, quaternary ammonium hydroxyl, optionally substituted alkyl, aryl, alkoxy, aryloxy, acyl, acyloxy, benzoyl or benzoyloxy, carboxylic acid or derivatives thereof, amido, imido, phosphate, phosphonate, sulphate, sulphonate, silicate, borate and sulphide.

In the process of the invention, the anhydride is subjected to a peroxidation, which cleaves at least one of the anhydride groups, to form one acid group and one peracid group. Where the anhydride is derived from two different acids groups that is which differ in the definition of $R^3$ or in the definition of X in the formula I, substituents in the groups attached to the X=O groups of the anhydride may be incorporated to affect the rate of hydrolysis of the group and/or to affect the likelihood of the anhydride cleaving at one or other side of the oxygen atom in the backbone. Substituents may thus affect the relative proportions of the two or more possible peracid formed in the "perhydrolysis" reaction. For instance where the anhydride is to be used as a bleach activator, and where the anhydride comprises units formed from a relatively hydrophilic and a relatively hydrophobic group, it may be desirable to affect by chemical substitution the ratio of relatively hydrophilic peracid and relatively hydrophobic peracid formed in the bleaching liquor.

In the present invention the term "bleaching composition" is exemplified by but not limited to detergents, especially laundry detergents, dishwashing detergents, hard surface cleaners, including kitchen and bathroom cleaners, toilet cleaners, pipe cleaners, dry bleaches, compositions for bleaching wood, paper and pulp bleaching compositions, fabric and fibre bleaching compositions and oral hygiene compositions including mouthwashes, toothpastes and plaque-removing compositions and depilatory creams. In such bleaching compositions, there is included a "bleach precursor" that is a composition which releases perhydroxyl groups ($OOH^-$), usually an organic or inorganic persalt, such as perborate, persulphate or percarbonate. Alternatively, bleaching compositions according to the invention may be used in conjunction with separate compositions which comprise such a bleach precursor. In these compositions a mixture of polyacid polyanhydrides may be used.

The polyanhydrides are of primary interest in the context of bleaching compositions. However, they may also be used as starting materials for processes in which they are peroxidised and in which the peracid product is subsequently used as an oxidising agent in contexts other than bleaching. For instance the products may be used as oxidising agents to form epoxides from ethylenically unsaturated hydrocarbons, in hydroxylation and other uses in synthetic chemistry, as PVC stabilisers etc. The peracid may also be used in free radical polymerisation, either as an initiator, or as an inhibitor. The peracid product may be provided in the form of salts, in particular alkali metal, alkaline earth metal (especially magnesium) or ammonium salts. The product peracid or salt may be recovered before being subsequently used, or may be produced in situ by the perhydrolysis reaction and used immediately.

Bleaching compositions also cover disinfecting compositions and other biocidal compositions for inhibiting the growth of bacteria, fungus, yeasts etc. The compositions may for instance be for application to plants as pesticides. As well as including bleach precursors which form peroxide, the anhydride compounds also act as activator for hypochlorite bleaches.

In the present invention we have found that the polyanhydrides have physical properties which make them particularly attractive for use as bleach activators or as starting materials for other processes in which they are subsequently perhydrolysed. The compounds may be liquids but are generally solids. For instance the polymeric anhydrides may be solids which are found to be less waxy and sticky than the monomeric cyclic anhydrides of the monomer units from which the polymers are formed. They are thus easy to handle in production processes for compositions, and are easy to formulate, for instance into bleaching compositions, especially particulate compositions such as detergent compositions. We have found that the solids are also extremely storage stable. For instance we have found that some of the polymeric anhydrides are stable even in the presence of inorganic persalts under normal storage conditions.

Liquid or solid polyanhydrides may be used in liquid compositions, such as liquid detergent compositions.

The anhydrides used in the present invention may be produced by known processes of reacting the corresponding acids or acid derivatives. For instance the acids may be reacted in a dehydrative coupling reaction, optionally using a coupling agent such as phosgene or diphosgene, as described in WO-A-9001005 or in the Macromolecules (1987) reference cited above or without any coupling agent. Alternatively the starting material may comprise an acyl chloride of one of the acids and a carboxylic acid, which are reacted by a melt dehydrochlorination reaction by a process as described in the 1987 Macromolecules reference cited above. Carboxylic acid starting materials may alternatively be reacted as a melt with ketene, for instance as described in DE-A-1804067 cited above. Acids may be melt condensed, by refluxing with a monobasic carboxylic acid anhydride, usually acetic anhydride, for instance as described in US-A-4886870 cited above. Alternatively the trimethylsilyl ester of one of the acids may be condensed with the acid chloride of the other acid, for instance as described in US-A-4868265 above. Alternatively an acid chloride may be mixed with an alkali metal or silver salt, the alkali metal chloride being precipitated and filtered off, as described in US-A-4,110,074.

Where the anhydride is formed from dibasic acid units, the starting materials may comprise the cyclic, monomeric anhydrides. Such anhydrides are subjected to a ring opening melt polymerisation, optionally with a catalyst, for instance stannous 2-ethylhexanoate, for instance by a process as described by Albertsson et al, op cit.

For the production of a polymeric anhydride, the reaction is continued until the degree of polymerisation has reached that desired. Usually it is preferred for the average number of units in chain to be at least 5, more preferably at least 10, and especially 20 or more. The number of monomer units in a chain is believed to affect the melting point of the polyanhydride. In general it is preferred for this to be above room temperature so that the polyanhydride remains a solid under conditions of storage, but lower than about 100°C, for instance lower than 80°C. In general the lower the melting temperature the more rapidly the polyanhydride will disperse into aqueous solution especially under laundry washing conditions when the temperature of the liquor is in the range 40 to 60°C.

The process for producing polyanhydride may include two steps or more, for instance in the first of which oligomeric or pre-polymer intermediates are formed, which are subsequently condensed to form longer units,

optionally in the presence of other reactants such as other monomer units having two or more carboxylic acid groups. The oligomer polymer may be reacted with reactants capable of introducing other functional groups either as sidechains on the polymer backbone.

Other processes may include using esters, amides, nitriles or aldehydes, or carboxylic acid derivatives as starting materials.

Where a mixed anhydride is produced in the invention the starting materials may be provided in two different forms, in order to ensure that an anhydride linkage in the product is derived from the two different acid starting materials. For instance one starting material may be in the form of an acid chloride, and the second, different starting material may be in the form of an acid or a salt. The starting materials are condensed in a dehydrochlorination reaction or with the elimination of a metal chloride salt, for instance as described above. As each of the starting materials is dibasic, preferably the two reactive groups of one compound are the same. For instance one of the starting materials comprises two acyl acid chloride groups and the other comprises two carboxylic acid groups, this will ensure that the different groups alternate along the polymer chain.

A suitable process for producing polyanhydrides of carboxylic acids is the condensation of carboxylic acid starting materials in solution of acetic anhydride. The molten acids are reacted with acetic anhydride at an elevated temperature, preferably above 50°C and preferably above the boiling point of the anhydride, so that the reaction is conducted under reflux until the desired degree of polymerisation is reached. The acetic anhydride is preferably used in a stoichiometric excess of acetic anhydride. Acetic acid byproduct and excess acetic anhydride are then preferably removed by distillation. The distillation is preferably conducted under vacuum and at a temperature which favours production of product of the desired molecular weight polyanhydride. The distillation may be conducted for instance at a constant temperature (90-120°C) and under gradually increasing vacuum (for instance in steps of about 50 mbar from 150 mbar down to 25 mbar) until effectively all the acid and excess acetic anhydride has been removed.

The process of reaction of the acids with acetic anhydride may alternatively be carried out in the presence of an inert organic solvent, such as toluene or xylene. Especially preferred is a solvent which codistills or azeotropes with acetic acid byproduct and/or unreacted acetic anhydride, to aid recovery of the product anhydride. Alternatively organic solvent may be added to the product mixture and used to drive off acid byproduct and excess anhydride.

The product anhydride may be recovered by processes including purification processes including reprecipitation, for instance from petroleum ether. Such processes are described in Macromolecules (1987), cited above. The product recovered may include byproducts for instance cyclic intra molecular anhydrides, which need not be removed as they may have advantageous properties in the composition. A solid polyanhydride product may be subjected to a process to alter the particle size, for instance to micronise it which can alter the reactivity/solubility in aqueous solution.

The particularly preferred use of the anhydrides is as a bleach activator, optionally in conjunction with other bleach activators, for instance tetraacetylethylenediamine (TAED), tetraacetylglycouril (TAGU), phthalic anhydride, succinic anhydride, isatoic anhydride or any of the other activators mentioned in the prior art referred to above. The activator may be included in a bleaching composition, especially a laundry bleaching composition such as a bleach booster or a complete detergent composition. Usually the composition also includes a peroxygen releasing compound, which can either be organic or, usually an inorganic persalt. Other components of the detergent or bleaching composition are conventional. Since the solid, especially the polymeric anhydrides are easy to handle in solid form, the anhydrides are of most use in granular detergent compositions.

The anhydride may be simply admixed direct in with the other detergent composition compounds. Alternatively it could be coformulated with builders (for example phosphates, polyphosphates, sodium carbonate, silicates, zeolites clays, borax), complexing or sequestering agents (ethylenediamine tetraacetic acid, diethylenepentamine pentaacetic acid, nitrilotriactic acid, citrates, phosphonates), enzymes, surfactants (linear alkylbenzene sulphonates, phenol sulphonates, alcohol ether sulphates, alcohol ethoxylates, amphoteric and cationic surfactants), soap, polyacrylates, hydrotropes, mineral oil and foam regulators.

The polyanhydride containing composition may contain byproducts of the reaction for forming it, for instance, including cyclic intra molecular anhydrides of the dibasic acid units from which the polymer is derived. These generally are not deleterious in bleaching compositions or in the other uses of the anhydrides covered herein.

It has been found that the polymeric anhydrides give particularly advantageous performance as bleach activators, even at low temperatures. It has been found that a copolymeric anhydride of sebacic and adipic acids is relatively stable in storage conditions even in the presences of sodium perborate, but still gives good peroxygen release characteristics at low temperatures in perborate -containing wash liquors.

The following examples illustrate the invention.

TECHNIQUES

1. PERFORMANCE TESTS

1.1 REAGENTS

| | |
|---|---|
| Stains: | Red Wine on cotton |
| Detergent Base: | IEC base - standard non-compact type containing OBA (Optical Brightening Agent) & Antifoam. |
| | WMP base (compact-type, phosphate-free) |
| | (Supplier Westlairds UK contents - see below Tables 1 & 2) |
| Perborate: | Sodium Perborate Tetrahydrate (PBS4) |
| Water Hardness: | Calcium Acetate (CaAc) to give 100 ppm in the wash |

| IEC base Detergent - COMPONENT | % |
|---|---|
| | |
| Linear alkylbenzene sulphonate (n=11.5) | 6.4 |
| Tallow alcohol ethoxylate (14 EO) | 2.3 |
| Sodium soap of mixed fatty acids | 2.8 |
| EDTA, tetra sodium salt | 0.2 |
| STPP | 35.0 |
| Sodium silicate | 6.0 |
| Magnesium silicate | 1.5 |
| CMC | 1.0 |
| Sodium sulphate | 16.8 |
| Water | 7.8 |
| Stilbene based optical brightener | 0.2 |
| | |
| TOTAL | 80 |

| WMP Base Detergent - COMPONENT | % |
|---|---|
| | |
| Linear alkylbenzene sulphonate (n=11.5) | 7.4 |
| $C_{12-18}$alcohol ethoxylate (7 EO) | 4.0 |
| Sodium soap (65% $C_{12-18}$, 35% $C_{20-22}$) | 2.8 |
| Zeolite A | 25.0 |
| Sodium Carbonate | 10.0 |
| Sodium salt of a Copolymerisate of Acrylic and Maleic acid (CP5) | 4.0 |
| Sodium silicate | 3.0 |
| CMC | 1.0 |
| EDTA | 0.2 |
| Stilbene based optical brightener | 0.2 |
| Sodium sulphate, Water | 18.8 |
| Protease enzyme prills | 0.5 |
| | |
| TOTAL | 77.0 |

1.2 APPARATUS

Wascator FOM 71MP washing machines - programmed to BS 4923 (HLCC) wash programmes (nominal 20 litre cold fill) 2-3kg of Polyester backing cloth per machine (approx. 50cm square)
HunterLab D25M Colorimeter with UV filter.

1.3 METHOD

1.3.1 Stain Swatch Preparation

Stained swatches 12cm x 12 cm are cut from bales of stained cloth, with tea or red wine stain.

1.3.2 Standardisation Procedure

5 washes at 40°C and 5 washes at 60°C are conducted on 4 red wine stained swatches per wash. Each wash uses a predetermined quantity (total weight 150 g including 22.5 g PBS4) of standard detergent formulation. The reflectance of the swatches is determined before and after the wash.
The percent stain removal is used using the following equation

$$\frac{R_A - R_B}{R_S - R_B} \times 100 = \%\ \text{Stain Removal}$$

$R_A$ = Reflectance of washed swatch
$R_S$ = Reflectance of standard unstained swatch
$R_B$ = Reflectance of unwashed swatch

1.4 WASH TEST PROCEDURE

The standardisation procedure is repeated, but using the bleaching composition under test in combination with the same quantity of basic detergent. In particular the test formulation contains 22.5 g PBS4, a predetermined quantity of the peracid precursor/activator compound (0.0263 moles) and the remainder up to 150 g of IEC detergent.

The new activators are compared to commercially available bleach activator tetraacetylethylene diamine

(TAED) (on the basis of equivalent theoretical peroxyacid release).

2. PERACID RELEASE TEST

2.1 Principle

The method is based on a standard iodometric reaction. A small sample of proxyacid is injected automatically into a reaction manifold of a flow injection analyser where it is mixed with potassium iodide under acidic conditions. Peroxyacids react with potassium iodide to produce $I^{3-}$. $I^{3-}$ gives a coloured species which has an absorbance peak maximum detected at a wavelength of approximately 400 nm. The sample passes through a spectrophotometer which detects the peak maximum. Analysis of the amount of peroxyacid release is calculated via dedicated computer.

2.2 Method

A predetermined quantity (3.5 x $10^{-3}$ moles per ℓ liquor) of the peracid precursor under test is added to 1.5 ℓ of distilled water containing 16.6 g of IEC detergent base, 3 g sodium perborate tetrahydrate, 2 drops wetting agent (Lutensit A-LBA, trade mark) and 1 drop anti foam thermostatically controlled at a predetermined temperature. The solution is stirred. The flow injection analyser automatically takes samples at 1 minute intervals over the period of about 30 minutes and reacts them with a 10% w/w potassium iodide solution and glacial acetic acid. The end point of the titration is determined automatically from the values of the absorbance at 400 nm recorded by the analyser.

2.3 Results

The quantity of peracetic acid released after a given period from addition of activator is determined from a standard curve set up by injecting solutions containing known amounts of the peracetic acid.

3. DETERMINATION OF EQUIVALENT WEIGHT OF ANHYDRIDE

3.1 In order to determine the equivalent weight of an anhydride product, that is the weight per anhydride unit the following technique is used. It is based on reacting the anhydride with an excess of morpholine and back-titrating the residual morpholine with hydrochloric acid.

3.2 A weighed amount (0.5-1 g or more if necessary) of the dry product is placed in a conical flask, to which is then added 25 ml of a 0.5 M solution of morpholine in methanol. The mixture is allowed to react for about 15 minutes. One drop of an indicator solution (comprising 1.0 g methyl yellow and 0.1 g methylene blue) is added to the solution which is then titrated by a solution of 0.5 M hydrochloric acid in methanol. The end point of the titration occurs at the colour change green to amber. The titre is TiMl. A blank titration is conducted on 25 ml of the morpholine solution in the absence of any anhydride to give a titre. A similar titration is conducted on a known quantity of acetic anhydride to give a titre $T_3$.

3.3 The result is calculated from the following formula:

$$\text{Equivalent weight} = \frac{1000 \times \text{weight of solid product}}{(T_2 - T_1) \times F}$$

where F is a factor representing the weight of acetic anhydride of known purity per mole of anhydride units determined from the titration of acetic anhydride. It is calculated by the following formula:

$$F = \frac{\text{weight of acetic anhydride} \times 102.09}{(T_2 - T_3)}$$

4. Molecular weight determination

4.1 Gel permeation chromatography is used to determine the molecular weight of the polyanhydride products under the following conditions:

| | | |
|---|---|---|
| Column | - | PL-Gel-10 Mix, 300mm x 7.5mm (supplied by Polymer Labs). |
| Pump | - | Bruker LC2200 |
| Detectors | - | Knauer R.I.model 198; Bruker/Knauer U.V. model 731 (detecting at 254nm). |
| Eluent | - | Dichloromethane |
| Flow Rate | - | 1.0 $ml.min.^{-1}$. |
| Software | - | "Chromatography Master" by Epson Ver. 5.2/02. |
| Calibration | - | PL polystyrene standards 162 - 1,030,000 daltons. |

5. FABRIC DAMAGE

It is intended that the test represents undissolved detergent adhering to clothing during prolonged soaking. Woven woollen cloth, treated by the Hercosett process and undyed, was obtained from the Society of Dyers and Colourists, Bradford, West Yorkshire.
The test detergent formulation plus PBS4 is enclosed in a "pouch" of wool which is left to soak for 16 hours in water at room temperature. After rinsing and drying the pieces were examined by eye and ranked on a scale of 0 to 5, higher numbers being worse according to the number of raised yellow spots present. These are areas which have been irreversibly damaged by peroxygen species.

5.2 DYE DAMAGE

A range of dyes, each used to dye individual swatches of cotton cloth, have a layer of a powder composition including one of the two base detergents including PBS4 and the activator under test applied and are then soaked with water and left for 18 hours. The swatches are then rinsed and dried. The change in dye colour after rinsing and drying is judged by eye and any damage noted on a scale of 0 to 5.

Example 1

Preparation of Poly (adipic anhydride)

1.1 Total acid:anhydride ratio 1:2.5

Adipic acid (58.4 g, 0.4 mole) was refluxed with acetic anhydride (102.1 g, 1.0 mole) for a period of four hours. The mixture was then allowed to cool to room temperature overnight. The excess acetic anhydride used, and acetic acid formed, was then stripped from the product mixture by vacuum distillation. When cool 100 $cm_3$ of toluene was added to the residue and stripped, under vacuum, on the rotary evaporator. The residue was allowed to cool to room temperature and solidify. The solid mass, so formed, was ground and sieved to a particle size of below 1000 microns.

Weight of solid isolated = 64.0 g
Melting Point: 66-67°C
IR Spectrum:
C=O bands at 1810 & 1740 $cm^{-1}$ the former of which is the more intense. This is consistent with an aliphatic acyclic anhydride.
Theoretical Equivalent Weight = 128.13
Actual Equivalent Weight = 132.97
1.2 The method was repeated but varying the ratio of adipic acid acetic anhydride in the range 1:10-1:1.5.

Example 2

Preparation of Poly (adipic - sebacic anhydride) 1:1 molar

Adipic acid (29.2 g, 0.2 mole) and sebacic acid (40.5 g, 0.2 mole) was refluxed with acetic anhydride (102.1 g, 1.0 mole) for a period of four hours. The mixture was then allowed to cool to room temperature overnight. The excess acetic anhydride used, and acetic acid formed, was then stripped from the product mixture by vacuum distillation. The crude product was treated with 40-60 petroleum ether and the mixture stripped of solvent, under vacuum, on the rotary evaporator. The residue was allowed to cool to room temperature and solidify. The solid mass, so formed, was ground and sieved to a particle size of below 1000 microns.

Example 2.1 Weight of solid isolated = 59.7 g

Melting Point: 56-58°C
IR Spectrum:
C=O bands at 1810 & 1740 $cm^{-1}$ the former of which is the more intense. This is consistent with an aliphatic acyclic anhydride.
GPC results: Mn 893.5, Mw=3039.0, PD 3.401
Theoretical Equivalent Weight for a polymer of 10 units in length = 151.3
Actual Equivalent Weight = 158.73

Example 2.2-2.4

In another run the equivalent weight of the solid was 272, and in another (example 2.3) was 181 indicating varying amounts of acid starting material remained in the product.

The example was repeated (example 2.4) but using a 1:1 mole total acid:acetic anhydride starting materials. The equivalent weight of solid product was 3/7 indicating that relatively large quantities of the dibasic acid starting materials remained in the product.

The example was repeated but using acid:anhydride ratios of 1:2 and 1:5. The following results were obtained:

Example 2.5 (acid:anhydride 1:2)

equivalent weight = 237.8

Example 2.6 (acid:anhydride 1:5)

equivalent weight = 272.0

Example 3

Preparation of Poly (adipic-sebacic anhydride)

3.1 Adipic:sebacic 2.1 molar

Adipic acid (43.0 g, 0.3 mole) and sebacic acid (30.3 g, 0.15 mole) was refluxed with acetic anhydride (114.9 g 1.125 mole) for a period of four hours. The mixture was then allowed to cool to room temperature overnight. The excess acetic anhydride used, and acetic acid formed, was then stripped from the product mixture by vacuum distillation at a temperature of 140°C. The residue was allowed to cool to room temperature and solidify. The solid mass, so formed, was ground and sieved to a particle size of below 1000 microns.
Weight of solid isolated = 82.5 g
Melting Point: 47.5°C
IR Spectrum:
C=O bands at 1810 & 1740 $cm^{-1}$ the former of which is the more intense. This is consistent with an aliphatic acyclic anhydride.
Theoretical Equivalent Weight for a polymer of 10 units in length = 142.8
Actual Equivalent Weight = 143.2

3.2 Adipic:sebacic 1:2 molar

Example 3.1 was repeated but using 18.0 g adipic acid (0.13 mole) and 54.6 g sebacic acid (0.27 mole).
Weight of solid isolated 50.3 g
Theoretical equivalent weight for a polymer 10 units in length = 159.8
Actual equivalent weight = 175.4

Example 4

Preparation of poly (sebacic anhydride)

Sebacic acid (81.0 g, 0.4 mole) was refluxed with acetic anhydride (102.0, 1.0 mole) for a period of 4 hours. The mixture was then allowed to cool to room temperature overnight. The excess acetic anhydride used, and acetic acid formed, was then stripped from the product mixture by vacuum distillation at a temperature of 140°C. The residue was allowed to cool to room temperature and solidify. The solid mass, so formed, was ground and sieved to a particle size of below 1000 microns.

Weight of crude solid isolated = 87.0 g

The solid was recrystallised from petroleum ether and dried under vacuum to give 7.0 g solid. The solid had a melting point of 79°C. The equivalent weight was 186.5 (theoretical equivalent weight for a polymer 10 units in length = 176.8).

Example 5

Preparation of poly (adipic succinic anhydride)

5.1 (1:1 molar)

Adipic acid 136.5 g,l 0.25 mole) and succinic acid (29.5 g, 0.25 mole) was refluxed with acetic anhydride (127.5 g, 1.25 moles) for a period of 4 hours.
was then allowed to cool to room temperature overnight. The excess acetic anhydride used, and acetic acid formed, was then stripped from the product mixture by vacuum distillation at a temperature of 140°C. The residue was allowed to cool to room temperature and solidify. The solid mass, so formed, was ground and sieved to a particle size of below 1000 microns.

The equivalent weight of the recovered solid was 187.1 compared to a theoretical equivalent weight of 114.0 imdicating the presence of acid starting materials. It is also expected that the product will include monomeric intramolecular cyclic succinic anhydride, the presence of which should be determined by nmr and/or gel permeation aronatography.

5.2 Molar ratio 10:1

Adipic acid (29.2g, 0.2 M), succinic acid (2.36 g, 0.02 M) and acetic anhydride (56.1 g, 0.55 M) were refluxed together for a period of 4.5 hours. The mixture was then stripped at reduced pressure to remove all acetic anyhydride and acetic acid. The residue, so formed, solidified on cooling.

Wt. of product = 28.1 g

M.Pt = 68°-70°C

Equivalent weight by morpholine titration = 131.7

Theoretical value for a polymer of ten units in length = 123.4

IR Spectrum:

C=O bands for anhydride groups at 1814 and 1750 $cm^{-1}$ absence of C=O bands at 1870 $cm^{-1}$ indicates absence of cyclic anhydrides.

HPLC analysis (by area %) indicated that the product contained 5% by weight succinic anhydride, thus 70% of the succinic acid has been incorporated into the polymer material.

Example 6

Effect of varying the reflux time

Reaction mixture with the quantities of materials used in Example 2 were made up. The reactants were then refluxed (average temperature 142.8°C), each for a different period (1 to 16 h) after which the product was worked up by the same technique, ie by distilling at 100°C under a 27 in Hg vacuum until distillation ceases. The equivalent weight and physical properties of the product were assessed. The results are in the following table.

Table 1

| Example | Reflux time h | Equivalent weight | "feel" or melting point |
|---|---|---|---|
| 6.1 | 1 | 167.3 | Tacky |
| 6.2 | 2 | 163.4 | Tacky |
| 6.3 | 6 | 175.0 | 60.4 |
| 6.4 | 16 | 194.0 | 65-71 |

From these results it appears that optimum reflux time is 4-4½ hours after which degradation starts and formation of dibasic acid anhydride ends.

Example 7

Preparation of Poly(Adipic Glutaric Anhydride)

Adipic acid (86.4 g, 0.59 M), glutaric acid (14.4 g, 0.11 M) and acetic anhydride (178.5 g, 1.75 M) were refluxed together for a period of 4 hours. The mixture was then stripped at reduced pressure to remove all acetic anyhydride and acetic acid. The residue, so formed, solidified on cooling.

Wt. of product = 110.0 g

M.Pt = 46°-48°C

Equivalent weight by morpholine titration = 125.2

Theoretical value for a polymer of ten units in length = 123.8

IR Spectrum:

C=O bands for anhydride groups at 1814 and 1750 $cm^{-1}$ absence of C=O bands at 1870 $cm^{-1}$ indicates absence of cyclic anhydrides.

Example 8

Reaction of disodium adipate and acetyl chloride

To sodium hydroxide (16.4 g, 0.41 mole) in 60 ml water was added adipic acid (29.2 g, 0.2 mole). The reaction to form the disodium salt was noted to be exothermic. The product was precipitated by addition of methanol and solid recovered.

Disodium adipate (19.0 g 0.1 mole) acetyl chloride (31.4 g, 0.4 mole) were heated in Perklone (1,1,2,2,tetrachloroethylene) at 70°C for 24 h.

The product may be used as starting material to react with other dibasic acids to form polyanhydrides.

Example 9

Preparation of Poly(adipic anhydride) in the presence of organic solvent

Adipic acid (20.0 g, 137 mM) was refluxed with acetic anhydride (28.0 g, 274 mM) in 250 $cm^3$ of toluene for 5 hours. The reaction mixture was concentrated under vacuum and the residue was extracted into dichloromethane. After filtering, the solvents were removed under vacuum to give the polymer as a white powder.

Wt. of product = 17.2 g

Equivalent weight by morpholine titration = 130.08

Theoretical value for polymer of ten units in length = 125.8

IR Spectrum:

C=O bands at 1814 and 1744 $cm^{-1}$ the former of which is the more intense. This is consistent with an aliphatic acyclic anhydride. The absence of COOH groups is indicated by the absence of the appropriate absorption bands.

GPC results:

Using the cited method.

$M_n$ = 1270

$M_w$ = 2800

PD = 2.2

$^1$H N.M.R. spectrum:

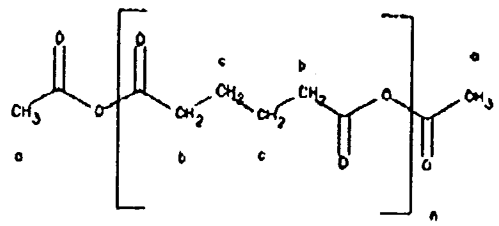

1.7 ppm - $H_c$

2.2 ppm - $H_a$

2.5 ppm - $H_b$

The average polymer size can be estimated from the ratio of protons in the polymer backbone : protons in the acetyl end-groups. For this particular product the formula is :

8n:6 (where n = no. of repeating units)

The ratio of the appropriate integrations from the N.M.R. spectrum gives an estimate of the average polymer length. In this particular case, n was found to be 9.84, i.e. the polymer molecule contain 9.84 adipic units on average. Assuming that the product contains acetyl end-groups only, the average Molecular weight = 1363.

## Example 10

### 10.1 Preparation of Disuccinoyl Hexamethylene Glycol

(5,12-dioxa-4,13-dioxo-1,16-hexadecanedioic acid) 1,6-Hexanediol (20.0 g, 0.169 M) and succinic anhydride (33.9 g, 0.338 M) were melted together at 120°C and stirred for 30 minutes to complete dissolution. The resulting crude product was recrystallised from acetone.

Wt. of product = 37.0 g (purity 87.6% by HPLC)

IR Spectrum:

C=O band for ester groups at 1729 $cm^{-1}$

C-O band for ester groups at 1189 and 1174 $cm^{-1}$

C=O band for carboxylic acid groups at 1709 $cm^{-1}$

### 10.2 Polymerisation of Disuccinoyl Hexamethylene Glycol

Disuccinoyl hexamethylene glycol (30.0 g, 94.2 mM) and acetic anhydride (24.0 g, 235 mM) were refluxed together in THF for 135 minutes. The mixture was left to cool overnight. The solvent was removed under vacuum to give a pale yellow residue, which solidified to a white waxy solid on standing.

Yield = 30.5 g

Equivalent weight by morpholine titration = 285.0

Theoretical value for a polymer of ten units in length = 282.3

IR Spectrum:

C=O bands for anhydride groups at 1818 and 1790 $cm^{-1}$

C=O bands for ester groups at 1733 $cm^{-1}$

C-O bands for ester groups at 1209 and 1185 $cm^{-1}$

The absence of bands between 3200-2800 $cm^{-1}$ indicates the absence of -OH groups.

## Example 11

### Preparation of Poly (3,6,9-Trioxaundecanedioic Anhydride)

3,6,9-Trioxaundecanedioic acid (50.0 g, 0.23 M) and acetic anhydride (115.0 g, 1.13 M) were refluxed together for 5 hours. The mixture was then stripped under reduced pressure to remove acetic anhydride and acetic acid, to leave an amber liquid residue.

Yield = 54.5 g

Equivalent weight by morpholine titration = 226.5

Theoretical value for a polymer of ten units in length = 194.9
IR Spectrum:
C=O bands for anhydride groups at 1834 and 1761 $cm^{-1}$

Example 12

Preparation of Poly(Adipic 1,2,3,4-Butanetetracarboxylic Anhydride)

Adipic acid (150.0 g, 1.03 mol), 1,2,3,4-butanetetracarboxylic acid (20.0 g, 0.09 mol) and acetic anhydride (305.0 g, 3.0 mol) were refluxed together for a period of 5 hours. The mixture was then stripped at reduced pressure to remove all acetic anhydride and acetic acid. The residue, so formed, solidified on cooling.

Wt of product = 181.8 g
M.Pt = 72°-74°C
Equivalent weight by morpholine titration = 135.6
Theoretical value for a polymer of infinite length = 124.0
IR Spectrum:
C=O bands for anhydride groups at 1808 and 1743 $cm^{-1}$

Example 13

Preparation of Poly(Adipic Citric Anhydride)

Citric acid (19.2 g, 0.1 mol), adipic acid (146.0 g, 1.0 mol) and acetic anhydride (352.0 g, 3.45 mol) were heated together at 70°C for 5 hours. The mixture was stripped at reduced pressure to remove acetic acid and acetic anhydride. The residue solidified on cooling.

Wt of product = 168.5 g
Equivalent weight by morpholine titration = 125.2
Theoretical value for a polymer = 125.8
(assuming all COOH groups were converted to anhydride)
IR Spectrum:
C=O bands for anhydride groups at 1834 and 1761 $cm^{-1}$.

Example 14

14.1 Preparation of (4-Carboxy-N-Phthalimidyl)-6-aminohexanoic Acid

1,2,4-Benzentricarboxylic anhydride (96.0 g, 0.467 M), 6-aminohexanoic acid (65.6 g, 0.50 M) and triethylamine (8.0 g, 0.08 M) (as base catalyst) were added to 500 $cm^3$ toluene. The mixture was heated to, and maintained at reflux until a total of 9 $cm^3$ of water had been removed by azeotropic distillation. The mixture was allowed to cool and the white precipitate so formed was filtered off and dried. The product was found to be 96.6% pure by HPLC.

M.Pt = 187.5 -189.5°C

14.2 Preparation of Poly[(4-Carboxy-N-Phthalimidyl)-6-Aminohexanoic Anhydride]

(4-Carboxy-N-phthalimidyl)-6-aminohexanoic acid (30.5 g, 0.1 mol) and acetic anhydride (102.0 g, 1.0 mol) were refluxed for 5 hours. The mixture was then stripped under reduced pressure to remove acetic acid and acetic anhydride. The residue solidified on cooling.

Wt. of product = 33.9 g
Equivalent weight by morpholine titration = 325
Theoretical value for a polymer of ten units in length = 270.4

Example 15

Fractional Crystallisation of Poly(Adipic Anhydride)

Poly(adipic anhydride) and toluene were heated to, and held at reflux until all the solid had been dissolved. The solution was then held at a temperature of 55°C, by means of a water bath. The temperature of the solution

was allowed to fall, 1°C at a time. Solid began to precipitate at 48°C, and the temperature of the bath was adjusted to maintain a constant solution temperature of 48°C. After a period of 90 minutes the supernatant was removed, and the solid residue isolated and dried (Fraction A). The supernatant liquid was stripped to dryness, to yield more solid (Fraction B).

Starting poly(adipic anhydride)

Wt of solid = 80.0 g (M.Pt - 67°-69°C)
Morpholine Equivalent = 141.7
GPC results:
Weight average MW = 6531 PD = 3.571

Fraction A

Wt of solid = 53.3 g (M.Pt - 73°-75°C)
Morpholine Equivalent = 172.4
GPC results:
Weight average MW = 1710 PD = 2.034

Fraction B

Wt of solid = 23.2 g (M.Pt - 90°-101°C)
Morpholine Equivalent = 232.9
GPC results:
Weight average MW = 1531 PD = 1,609

Example 16

Poly(Adipic Anhydride) with COOH end-groups

A mixture of adipic acid 73.0 g, 05. mol) and acetic anhydride (51.0 g, 0.5 mol) were refluxed together for 4 hours. The resulting solution was treated with dichloromethane to remove the unreacted adipic acid. The solvent was removed and the residue so formed was treated with toluene and stripped to remove the acetic acid and acetic anhydride present.

Wt of solid = 23.4 g
M.Pt = 46°-48°C
Equivalent weight by morpholine titration = 175.0
Theoretical value for a polymer of ten units in length = 118.1
IR Spectrum:
C=O bands for anhydride groups at 1810 and 1745 $cm^{-1}$
C=O bands for COOH group at 1700 $cm^{-1}$
O-H bands at 3600 -2400 $cm^{-1}$ show the presence of COOH groups
GPC Results: Weight average MW = 2643 PD = 2.888
NMR Results:
$^{1}H$ NMR
2.15 ppm signal corresponds to $CH_2$ adjacent to COOH
3.0 ppm signal corresponds to $CH_2$ adjacent to anhydride group
12.0 ppm weak signal corresponds to H from COOH end-group.
$^{13}C$ NMR
169 ppm signal corresponds to C from anhydride group
174 ppm signal corresponds to C from COOH end-group

Example 17

Wash Tests

Wash tests as described above were conducted using some of the compounds prepared in the preparation Examples 1, 3, 4 and 6 as bleach activator. The amount of product that was used is reported in the table below.

The amounts of compounds of examples 1, 3 and 4 were selected on the basis of that one equivalent of activator released one mole of peracid. The amount of the compounds of examples 6.1, 6.2, 6.3 and 6.4 used were used at a slightly higher rate but all were of the same weight as each other. The compounds were compared to TAED of the same equivalent amount (i.e. the amount in theory able to produce the same amount of peracid) which in these experiments was 3.0 g.

Table 3

| Example | Weight used g | % stain removal as % of TAED - stain removal | |
|---|---|---|---|
| | | 40 | 60 |
| 1.1 | 3.498 | 101.4 | 97.7 |
| 3.2 | 4.609 | 100.2 | 98.9 |
| 4. | 4.897 | 95.6 | 97.9 |
| 6.1 | 4.110 | 94.0 | 98.1 |
| 6.2 | 4.110 | 93.7 | 97.8 |
| 6.3 | 4.110 | 95.6 | 96.7 |
| 6.4 | 4.110 | 99.9 | 99.1 |

*The results of the 60°C wash tests on the 4 samples using this activator have not been averaged because of the relatively high scatter.

The above results indicate that the new compounds have good performance in both low and high temperature wash tests.

Further tests were conducted about 2 weeks later on the product of example 4, the product having been stored at ambient temperature in a closed vessel in the meantime. The results were very similar to the first series of tests indicating no degradation of performance on storage.

Example 18

Peracid Release Tests

Some of the compounds produced in the previous examples were tested for their peracid release properties by the method set out above.

The table below shows the results in terms of the maximum amount of peracid released during the 30 min. test per mole of anhydride used.

Table 4

| Product of: | COMPOUND | Max. Release @ 40°C | | Max. Release @ 60°C | |
|---|---|---|---|---|---|
| | | Mol. | Mins. | Mol. | Mins. |
| Example 1 | Poly(adipic anhydride) | 0.07 | 30 | 0.36 | 2 |
| Example 2.1 | Poly(adipic - sebacic anhydride) [1:1] | 0.15 | 30 | 0.45 | 3 |
| Example 3.1 | Poly(adipic - sebacic anhydride [2:1] | 0.13 | 30 | 0.50 | 3 |

Example 19

19.1 Larger Scale Poly(adipic anhydride) Production

Adipic acid (1474 g, 10.0 mol) and acetic anhydride (2684.3 g, 26.3 mol) were heated to, and maintained

at reflux for a period of 4 hours. The mixture was then stripped at reduced pressure to remove the acetic acid and acetic anydride present.

The weight of product collected was 1566.2 g and its equivalent weight was found to be 127.4 and its melting point 62-64°C.

Samples of the solid product were, after recovery, subjected to grinding by hand and sifting to produce particles with diameters under 1000μm or to miconising to produce particles with diameters under 150μm.

19.2 Larger Scale Poly(sebacic anhydride) Production

The method described for example 4 was repeated but using 202.3 g sebacic acid and 255.2 g acetic anhydride as starting materials. The weight of product was 148.5 g and it had an equivalent weight of 187.0 and a melting point of 62-64°C.

19.3 Larger Scale 1:1 Adipic:Sebacic Polyanhydride Production

The method of example 2 was repeated but using 584 g (4 mole) adipic acid, 809 g (4 mole) sebacic acid and 2050 g (20 mole) acetic anhydride, the reflux time being 4 hours and the acetic acid and acetic anhydride being removed at 100°C and 25 mbar.

19.4 Performance Data - Stain Removal

The products of examples 19.1-3 were tested by the stain removal technique described above using IEC base detergent to give the results shown in Tables 5.1 and 5.2 below. The results reported in 5.1 and 5.2 were conducted on separate occasions.

Table 5.1

| Example | Acid from which Polymer was made | Weight used (g) | Red Wine | | Tea | |
|---|---|---|---|---|---|---|
| | | | 40°C | 60°C | 40°C | 60°C |
| 19.1 | Adipic acid[1] | 3.8846 | 74.6 | 85.2 | 45.8 | 61.1 |
| 19.1 | Adipic acid[2] | 3.8846 | 72.0 | 84.6 | 41.8 | 63.1 |
| 19.2 | Sebacic acid | 4.8552 | 75.7 | 84.1 | 43.8 | 61.6 |
| | None | - | 74.8 | 83.3 | 36.9 | 54.0 |

Table 5.2

| Example | Acid from which Polymer was made | Weight used (g) | Red Wine | | Tea | |
|---|---|---|---|---|---|---|
| | | | 40°C | 60°C | 40°C | 60°C |
| 19.3 | Adipic:sebacic [1:1] | 2.7368 | 73.9 | 82.3 | 39.8 | 52.6 |
| | None | - | 74.9 | 82.9 | 30.1 | 42.9 |

[1] - signifies micronised (≤ 150 micron)

[2] - signifies hand ground (≤ 1000 micron)

The results show that the polyanhydrides all give good bleaching activity especially on tea stains.

19.5 Fabric and Dye Damage

The products of examples 19.1 and 19.3 were tested for their fabric/dye damage tests described above to give the results shown in Tables 6 and 7.

Table 6 - With IEC Base

| Example | Activator | Reactive Red | Immedial Green | Immedial Black | White Cotton | Hercoset Wool |
|---|---|---|---|---|---|---|
| | None | 0 | 0 | 0 | 0 | 0 |
| | TAED | 5 | 3 | 2-3 | 0 | 0 |
| 19.1 | Adipic | 5 | 3 | 4 | 0 | 2 |
| 19.3 | Adipic sebacic | 4-5 | 2 | 5 | 0 | 0 |

Table 7 - With WMP Base

| Example | Activator | Reactice Red | Immedial Green | Immedial Black | White Cotton | Hercoset Wool |
|---|---|---|---|---|---|---|
| | None | 0 | 0 | 0 | 0 | 0 |
| | TAED | 5 | 4 | 4 | 0 | 4-5 |
| 19.1 | Adipic | 5 | 4 | 5 | 0 | 2 |
| 19.3 | Adipic sebacic | 5 | 4-5 | 4-5 | 0 | 1 |

Damage assessed visually: 0 = least damage, 5 = worst damage

Example 20 - Peracid Release Tests

Some of the polyanhydrides produced in previous examples were subjected to the above described peracid release test. The results are shown in table 8.

Table 8

| Product of example | Acid from which polymer was made | @ 20°C | | @ 40°C | | @ 60° C | |
|---|---|---|---|---|---|---|---|
| | | mol/mol | $Rel_{max}$ (min) | mol/mol | $Rel_{max}$ (min) | mol/mol | $Rel_{max}$ (min) |
| 19.1 | Adipic | 0.31 | 30 | 0.54 | 26 | 0.44 | 8 |
| 19.3 | Adipic - sebacic | - | - | 0.20 | 30 | 0.61 | 3 |
| 19.2 | Sebacic | 0.11 | 30 | 0.24 | 30 | 0.27 | 12 |
| 5.2 | Adipic - succinic | - | - | 0.50 | 15 | 0.32 | 7 |
| 7 | Adipic - glutaric | - | - | 0.19 | 30 | 0.80 | 2 |
| 10 | 5,12-dioxa-4,13-dioxo-1,16-hexadecanedioic | - | - | 0.09 | 30 | 0.70 | 2 |
| 11 | 3,6,9-Trioxaundecanedioic | 0.34 | 12 | 0.24 | 3 | - | - |
| 12 | Adipic - 1,2,3,4-Butanetetracarblxylic | - | - | 0.39 | 18 | 0.59 | 7 |
| 13 | Adipic - citric | - | - | 0.09 | 30 | 0.91 | 2 |
| 14.2 | (4-Carboxy-N-phthalimidyl)-6-aminohexanoic | - | - | 0.16 | 30 | 0.09 | 8 |

Example 21 - Relative Rates of Hydrolysis

Samples of poly(adipic anhydride), poly(sebaccic anhydride) product were made by the techniques described above and further products were made by similar techniques but using fumaric acid in one instance and dodecanedioic acid in another. These were then tested for their relative rates of hydrolysis in solution at pH 10 and 60°C and compared to TAED, produced commercially. The testing is based on a standard acid/base titration which determines the amount of sodium hydroxide required to maintain a pH of 10 in the solution (of sodium auryl sulphate at 1.33 g/ℓ) added sodium hydroxide being needed as the acidic species is generated on hydrolysis. The results show that the relative rates of hydrolysis (per equivalent) are in the order
TAED >> poly(fumaric anhydride > poly(dodecanedioic anhydride) ≈ poly(adipic anhydride) > poly(sebacic anhydride). It is expected that these results may give a good indication of the stability of the compounds in detergent formulations.

**Claims**

1. A process in which a polyacid polyanhydride compound comprising at least 2 polyacid units per molecule is reacted with a peroxygen compound to cleave an anhydride linkage in aqueous solution to form a peracid compound and the peracid compound is used as an oxidising agent.

2. A process according to claim 1 which is carried out in the presence of the substrate to be oxidised by the peracid.

3. A process according to claim 2 in which the oxidation reaction is a bleaching reaction and in which the substrate is selected from fibres, fabrics, pulp and paper.

4. A process according to any preceding claim in which the polyanhydride compound is derived from polybasic acids are selected from carboxylic, sulphonic and phosphonic acids, and preferably include a carboxylic acid, more preferably consisting of units derived from polybasic carboxylic acids.

5. A process according to any preceding claim in which the polyanhydride is a homopolymer of one polyacid monomer, or is a copolymer of two or more polyacid monomers.

6. A process according to any preceding claim in which the polyanhydride polymer comprises on average at least 2, preferably at least 5 and less than 50, polyacid monomer units.

7. A process according to any preceding claim in which the polyanhydride has the general formula I:

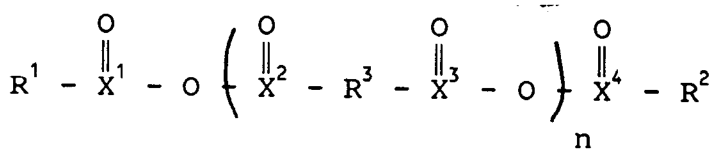 (I)

in which $R^1$ and $R^2$ are the same or different and each represents hydrogen, hydroxy, OM, halogen optionally substituted alkyl, optionally substituted alkenyl, optionally substituted alkynyl or optionally substituted aryl.

$X^1$ to $X^4$ are the same or different and each represents a carbon atom, a sulphur atom

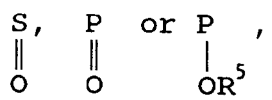

in which $R^5$ is hydrogen a metal ion or an optionally substituted alkyl group,

n is an integer of 2 or more,

the or each $R^3$ is independently selected from a bond, optionally substituted alkylene, optionally substituted alkenylene, optionally substituted alkynylene, optionally substituted arylene in any of which the $R^3$ group may be interrupted by a group selected from

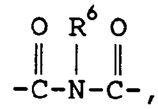, 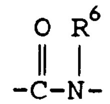, 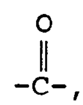,

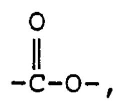, -O-, 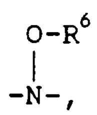, -S-, 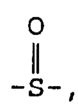, 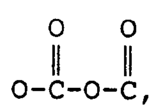, 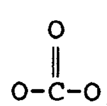,

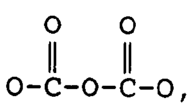, 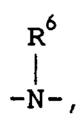, 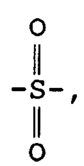, -S-O-, 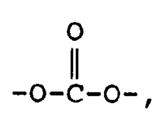, 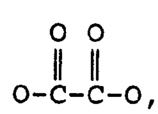,

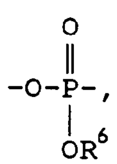, 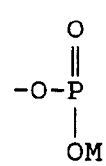, 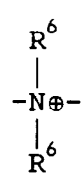

or a linear polyphosphate
in which the or each $R^6$ is independently selected from hydrogen, optionally substituted alkyl, optionally substituted alkenyl, optionally substituted alkynyl and optionally substituted aryl
the or each M is a monovalent metal ion.

8. A process according to claim 7 in which n is at least 5 and less than 50.

9. A process according claim 7 or 8 in which at least 1 of and preferably all of $X^1$ and $X^4$ are carbon atoms.

10. A process according to any of claims 7 to 9 in which at least 1 of the groups $R^3$ is a $C_{2-30}$-alkylene group.

11. A process according to any of claims 7 to 10 which includes at least two different $R^3$ groups.

12. A process according to any preceding claim in which the peroxygen compound is selected from hydrogen peroxide, inorganic salts and organic percarboxylic acids, and preferably an alkali metal perborate, -persulphate or -percarbonate.

13. A process according to any preceding claim in which the reaction is carried out at a pH of greater than 7, preferably in the range 7.5 to 10.5, more preferably 8.5 to 9.5.

14. Use of a polyacid polyanhydride compound comprising at least 2 polyacid units per molecule as an activator for a peroxygen bleach precursor.

15. Use according to claim 14 in which the peroxygen bleach precursor is hydrogen peroxide, inorganic salts and organic percarboxylic acids, and preferably an alkali metal perborate -persulphate or -percarbonate.

16. Use according to claim 14 or 15 in which the polyanhydride compound is as defined in any of claims 4 to 11.

17. Use according to any of claims 14 to 16 in a laundry, dishwashing or hard surface detergent or a pulp, paper or fabric bleaching process.

18. A bleaching composition comprising a polyacid polyanhydride compound comprising at least 2 polyacid units per molecule and optionally a bleach precursor.

19. A composition according to claim 18 in which the bleach precursor is hydrogen peroxide, inorganic salts and organic percarboxylic acids, and preferably an alkali metal perborate -persulphate or -percarbonate.

20. A composition according to claim 18 or 19 in which the polyanhydride compound is as defined in any of claims 4 to 11.